# EUROPÄISCHE PATENTSCHRIFT

(11) **EP 3 188 825 B1**
(45) Veröffentlichungstag und Bekanntmachung des Hinweises auf die Patenterteilung: **12.06.2019**
(21) Anmeldenummer: 15756895.7
(22) Anmeldetag: 27.08.2015
(51) Int. Cl.: B01J 2/26, C07C 29/76, C07C 35/12

(54) **VERFAHREN UND VORRICHTUNG ZUM HERSTELLEN VON L-MENTHOL IN FESTER FORM**
METHOD AND DEVICE FOR PRODUCING L-MENTHOL IN SOLID FORM
PROCÉDÉ ET DISPOSITIF DE FABRICATION DE L-MENTHOL SOUS FORME SOLIDE

(30) Priorität: 03.09.2014 DE 102014217603
(43) Veröffentlichungstag der Anmeldung: 12.07.2017
(73) Patentinhaber: IPCO Germany GmbH, 70736 Fellbach (DE)
(72) Erfinder: ROTH, Bernhard, 71563 Affalterbach (DE)
(74) Vertreter: Patentanwälte Ruff, Wilhelm, Beier, Dauster & Partner mbB
(86) Internationale Anmeldenummer: PCT/EP2015/069610
(87) Internationale Veröffentlichungsnummer: WO 2016/034481

(56) Entgegenhaltungen:
- EP-A1- 2 896 680
- EP-A2- 0 145 839
- WO-A1-2008/152009
- WO-A1-2012/007331

## Beschreibung

Die Erfindung betrifft ein Verfahren und eine Vorrichtung zum Herstellen von L-Menthol in fester Form.

Aus der internationalen Offenlegungsschrift WO 2008/152009 A1 ist bereits ein Verfahren zum Herstellen von L-Menthol in fester Form bekannt. Menthol ist ein natürlich vorkommender Wirkstoff, der in Pharmazie, Kosmetik und Lebensmittelindustrie breite Anwendung findet. In natürlichen Quellen, beispielsweise dem Pfefferminzöl, kommt Menthol in Form von vier diastereomeren Enantiomerenpaaren vor, von denen nur die Hauptkomponente, das (-)-Menthol oder L-Menthol, die gewünschten geschmacklichen und sonstigen sensorischen Eigenschaften hat. Von L-Menthol ist seit langem bekannt, dass es in vier verschiedenen Kristallmodifikationen erstarren kann, die bei gleicher chemischer Zusammensetzung unterschiedliche physikalische Eigenschaften aufweisen. Die Schmelzpunkte dieser verschiedenen Modifikationen liegen zwischen 33 °C bis 43 °C. Der Schmelzpunkt der stabilen alpha-Modifikation liegt bei 42 °C bis 43 °C. Generell weisen alle Feststoffe, die, wie L-Menthol, einen Schmelzpunkt nur knapp oberhalb der Umgebungstemperatur haben, eine starke Neigung auf, zu verbacken und zu verklumpen. Vorgeschlagen wird das Inkontaktbringen einer L-Menthol-Schmelze mit zwei voneinander beabstandeten, gekühlten Oberflächen. Solche gekühlte Oberflächen können beispielsweise aus endlosen Bändern aus glattem oder poliertem Edelstahl bestehen. Geeignet sind sogenannte Doppelbandkühler, um die L-Menthol-Schmelze gleichzeitig mit zwei gekühlten Oberflächen in Kontakt zu bringen. Vorgeschlagen wird weiter das Versetzen der L-Menthol-Schmelze mit Impfkristallen. Solche Impfkristalle können in einem sogenannten Kratzkühler erzeugt werden.

Aus der europäischen Offenlegungsschrift EP 0 145 839 A2 ist ein Tropfenformer zum Erzeugen von Tropfen einer fließfähigen Substanz bekannt. Der Tropfenformer weist eine rotierende, gelochte Außentrommel und eine an der Innenseite der Außentrommel anliegende, feststehende Düsenleiste auf. Mit dem Tropfenformer können Tropfen einer fließfähigen Substanz erzeugt und dann beispielsweise auf einem Kühlband abgelegt werden.

Aus der internationalen Offenlegungsschrift WO 2012/007331 A1 ist eine Vorrichtung zum Herstellen von Ammoniumnitrat enthaltenden Pastillen bekannt. Die Vorrichtung weist einen Tropfenformer mit einer rotierenden, gelochten Außentrommel und einer an der Innenseite der Außentrommel anliegenden, feststehenden Düsenleiste auf. Mit dem Tropfenformer wird eine Ammoniumnitrat enthaltende Produktschmelze vertropft und die so erzeugten Schmelzetropfen werden auf einem umlaufenden Kühlband abgelegt. Die Schmelzetropfen verfestigen während des Transports auf dem Kühlband und können im Bereich einer Umlenktrommel für das Kühlband abgenommen werden.

Die europäische Offenlegungsschrift EP 2 896 680 A1 beschreibt eine Vorrichtung zum Herstellen von Bitumenblöcken. Mittels eines Tropfenformers mit einer rotierenden, gelochten Außentrommel und einer an der Innenseite der Außentrommel anliegenden, feststehenden Düsenleiste werden Bitumentropfen aus geschmolzenem Bitumen erzeugt und auf einem unterhalb der Außentrommel angeordneten umlaufenden Kühlband abgelegt. Im Verlauf des Transports auf dem Kühlband verfestigen die Bitumentropfen zu Pastillen und können an einer Umlenktrommel des Kühlbands von diesem abgenommen werden.

Mit der Erfindung soll ein Verfahren und eine Vorrichtung zum Erzeugen von L-Menthol in fester Form bereitgestellt werden, wobei das hergestellte L-Menthol verbesserte Lagereigenschaften aufweist.

Erfindungsgemäß ist hierzu ein Verfahren zum Herstellen von L-Menthol in fester Form vorgesehen, bei dem folgende Schritte vorgesehen sind: Bereitstellen einer Menthol-Schmelze, Zuführen der Schmelze zu einem Tropfenformer mit einer rotierenden, gelochten Außentrommel und einer an der Innenseite der Außentrommel anliegenden, feststehenden Düsenleiste, Ablegen von mit dem Tropfenformer erzeugten Schmelzetropfen auf einem umlaufenden Kühlband, Verfestigen der Schmelzetropfen während des Transports auf dem Kühlband zu L-Menthol-Pastillen und Abnehmen der Pastillen im Bereich einer Umlenktrommel für das Kühlband.

Der Erfindung liegt die Erkenntnis zugrunde, dass glatte Oberflächen des L-Menthols für gute und über die Zeit gesehen stabile Lagereigenschaften sorgen. Weiter wurde von den Erfindern erkannt, dass durch Verfestigen von Schmelzetropfen erzeugte Pastillen allseitig vergleichsweise glatte Oberflächen aufweisen und insbesondere keinerlei Bruchkanten haben, so dass L-Menthol-Pastillen prinzipiell gute Lagereigenschaften haben sollten. Überraschenderweise ist es den Erfindern gelungen, mit einem Tropfenformer mit einer rotierenden, gelochten Außentrommel und einer an der Innenseite der Außentrommel anliegenden, feststehenden Düsenleiste L-Menthol-Pastillen von zufriedenstellender Qualität zu erzeugen, das bedeutet Pastillen, die der Kugelform angenähert sind und die dadurch im Verhältnis zum Volumen eine geringe Oberfläche aufweisen. Auch dadurch wird die Neigung zum Verklumpen während der Lagerung deutlich verringert. Den Erfindern ist es gelungen, die Tropfen der Menthol-Schmelze mittels des Tropfenformers mit einer ausreichenden Viskosität zu erzeugen, so dass die Tropfen beim Ablegen auf das Kühlband nicht auseinanderlaufen. Darüber hinaus ist es den Erfindern gelungen, durch einfaches Ablegen auf einem Kühlband mit lediglich einer gekühlten Oberfläche beim Verfestigen der Tropfen der Menthol-Schmelze auszukommen.

In Weiterbildung der Erfindung wird die Menthol-Schmelze durch einen Schabekühler vor dem Zuführen der Schmelze zu dem Tropfenformer zum Erzeugen von Impfkristallen geleitet. Vorteilhafterweise werden dabei mehr als 10 Gew.-% Impfkristalle in der Menthol-Schmelze im Schabekühler erzeugt. Insbesondere werden dabei mehr als 20 Gew.-% Impfkristalle in der Mentholschmelze im Schabekühler erzeugt. Vorteilhafterweise liegt eine Menge der Impfkristalle in der Mentholschmelze, die im Schabekühler erzeugt werden, zwischen 30 Gew.-% und 40 Gew.-%. Ein solcher Gewichtsanteil von Impfkristallen in der Menthol-Schmelze ermöglicht es, Tropfen der Menthol-Schmelze im Tropfenformer mit einer geeigneten Viskosität zu erzeugen, so dass diese Schmelzetropfen beim Aufkommen auf dem Kühlband nicht zu einem flachen Fladen auseinanderlaufen, sondern eine der Kugelform angenäherte Form behalten.

In Weiterbildung der Erfindung wird eine Temperatur an den Schabeflächen des Schabekühlers in einem Bereich zwischen 26 ° Celsius und 32 ° Celsius eingestellt.

Ein solcher Temperaturbereich an den Schabeflächen des Schabekühlers stellt nach den Erfahrungen der Erfinder den möglichen Temperaturbereich dar, in dem die Schabeflächen gehalten werden müssen, um zum einen die gewünschte Kristallkonfiguration in ausreichender Menge zu erzeugen und zum anderen ein Verstopfen des Schabekühlers durch zu große Kristallisationsmengen zu verhindern.

In Weiterbildung der Erfindung wird die Mentholschmelze in einem Kreislauf durch den Schabekühler gefördert, so dass die Mentholschmelze den Schabekühler mehrfach durchläuft.

Auf diese Weise können die zur Erzeugung von großen Mengen an Impfkristallen erforderlichen Verweilzeiten auf den Schabeflächen des Schabekühlers erreicht werden. Gleichzeitig wird die Strömungsgeschwindigkeit der in Breiform vorliegenden Mentholschmelze hoch genug gehalten, um ein Verstopfen des Schabekühlers durch zu starke Kristallisation zu verhindern.

In Weiterbildung der Erfindung beträgt eine Fördermenge im Kreislauf durch den Schabekühler mindestens das 10-fache, insbesondere das 20-fache, einer Fördermenge vom Schabekühler zum Tropfenformer.

Auf diese Weise lässt sich die gewünschte große Menge an Impfkristallen erzeugen und ein Verstopfen des Schabekühlers wird verhindert.

In Weiterbildung der Erfindung wird der Schabekühler mittels flüssigem Kühlmittel, insbesondere Wasser, gekühlt, wobei eine Kühlmittelmenge, die durch den Schabekühler gefördert wird, mindestens das 10-fache der Mentholschmelzenmenge beträgt, die durch den Schabekühler gefördert wird.

Durch eine im Verhältnis zur Mentholschmelzenmenge sehr große Kühlmittelmenge wird die Einstellung der Temperatur an den Schabeflächen des Schabekühlers im gewünschten Temperaturbereich sichergestellt.

In Weiterbildung der Erfindung erfolgt das Einstellen einer Temperatur des flüssigen Kühlmittels in einer Temperiereinheit unter Verwendung von heißer Kühlflüssigkeit oder Dampf und kalter Kühlflüssigkeit.

Auf diese Weise können Schwankungen der Temperatur der Kühlflüssigkeit sehr rasch ausgeglichen werden und es kann sowohl geheizt als auch gekühlt werden, um die Temperatur der Schabeflächen in dem gewünschten eng tolerierten Bereich zu halten.

In Weiterbildung der Erfindung erfolgt das Leiten der Mentholschmelze durch einen Vorkühler vor dem Zuführen der Schmelze zu dem Tropfenformer.

Auf diese Weise wird das Einstellen der gewünschten Viskosität der Mentholschmelze im Tropfenformer erleichtert.

In Weiterbildung der Erfindung erfolgt das Leiten der Mentholschmelze durch einen Schabekühler zum Erzeugen von Impfkristallen nach dem Leiten der Mentholschmelze durch den Vorkühler und vor dem Zuführen der Schmelze zu dem Tropfenformer.

Auf diese Weise wird das Erzeugen von Impfkristallen im Schabekühler erleichtert, da die Mentholschmelze bereits vorgekühlt in den Schabekühler strömt.

In Weiterbildung der Erfindung ist das Einstellen einer Temperatur im Tropfenformer oberhalb des Schmelzpunkts der Menthol-Schmelze vorgesehen, so dass eine Kristallisation der Menthol-Schmelze im Tropfenformer nur in sehr geringem Ausmaß erfolgt und Ablagerungen vermieden werden.

Ein solcher Einstellvorgang einer vordefinierten Temperatur im Tropfenformer ist für den Erfolg des erfindungsgemäßen Verfahrens wesentlich. Die Einstellung der Temperatur muss dabei mit geringen Toleranzen erfolgen. Hierzu sind die Düsenleiste des Tropfenformers umgebende Kanäle für ein Mittel zum Temperieren der Düsenleiste vorgesehen, vorteilhafterweise innerhalb eines einstückigen Blocks bzw. Kerns des Tropfenformers, in dem auch die Düsenleiste ausgebildet ist. Einerseits muss die Menthol-Schmelze innerhalb des Tropfenformers flüssig bzw. pastös gehalten werden, so dass sich keine Ablagerungen bilden. Auf der anderen Seite muss eine ausreichend hohe Viskosität der Menthol-Schmelze erzielt werden, so dass die erzeugten Tropfen beim Auftreffen auf das Kühlband nicht sofort zu Fladen auseinanderlaufen. Innerhalb des Tropfenformers und speziell im Bereich eines zentralen Zuführkanals und einer Düsenleiste des Tropfenformers muss eine Temperatur daher sehr exakt auf den gewünschten Temperaturbereich eingestellt werden.

Das der Erfindung zugrundeliegende Problem wird auch durch eine Vorrichtung zum Herstellen von L-Menthol in fester Form gelöst, die Mittel zum Bereitstellen einer Menthol-Schmelze, einen Tropfenformer mit einer rotierenden, gelochten Außentrommel und einer an der Innenseite der Außentrommel anliegenden feststehenden Düsenleiste sowie ein unterhalb der Außentrommel angeordnetes umlaufendes Kühlband aufweist, wobei stromaufwärts des Tropfenformers ein Schabekühler zum Erzeugen von Impfkristallen in der Menthol-Schmelze angeordnet ist.

In Weiterbildung der Erfindung weist der Schabekühler eine kreiszylindrische, gekühlte Innenfläche auf, die von auf einer rotierenden Welle angeordneten Schabern überstrichen wird.

Es hat sich gezeigt, dass solcher Art ausgebildete Schabekühler ausreichende Mengen an Impfkristallen erzeugen können und dennoch nicht zur Verstopfung neigen.

In Weiterbildung der Erfindung ist stromaufwärts des Schabekühlers ein Vorkühler für die Mentholschmelze angeordnet.

Das Vorsehen eines Vorkühlers erleichtert die gewünschte Erzeugung großer Mengen an Impfkristallen.

In Weiterbildung der Erfindung sind der Vorkühler, der Schabekühler und der Tropfenformer mit separaten Kühlflüssigkeitskreisläufen versehen.

Auf diese Weise wird das Einhalten eng tolerierter Temperaturbereiche im Vorkühler, im Schabekühler und im Tropfenformer sichergestellt.

In Weiterbildung der Erfindung sind den Kühlkreisläufen jeweils separate Temperiereinheiten zugeordnet, wobei jede Temperiereinheit eine Heizvorrichtung zum Bereitstellen von heißer Kühlflüssigkeit oder Dampf und eine Kühlvorrichtung zum Bereitstellen von kalter Kühlflüssigkeit aufweist.

Mittels solcher Temperiereinheiten kann die Temperatur der Kühlflüssigkeit sehr schnell geregelt werden und in einem engen Temperaturbereich gehalten werden.

Weitere Merkmale und Vorteile der Erfindung ergeben sich aus den Ansprüchen und der folgenden Beschreibung bevorzugter Ausführungsformen der Erfindung im Zusammenhang mit den Zeichnungen. Einzelmerkmale der beschriebenen und dargestellten Ausführungsformen lassen sich dabei in beliebiger Weise miteinander kombinieren, ohne den Rahmen der Erfindung zu überschreiten. In den Zeichnungen zeigen:
- Fig. 1: eine schematische Darstellung einer erfindungsgemäßen Vorrichtung zum Herstellen von L-Menthol in fester Form;
- Fig. 2: eine vergrößerte Darstellung des Tropfenformers der Vorrichtung der Fig. 1; und
- Fig. 3: eine schematische Darstellung einer erfindungsgemäßen Vorrichtung zum Herstellen von L-Menthol in fester Form, gemäß einer weiteren Ausführungsform.

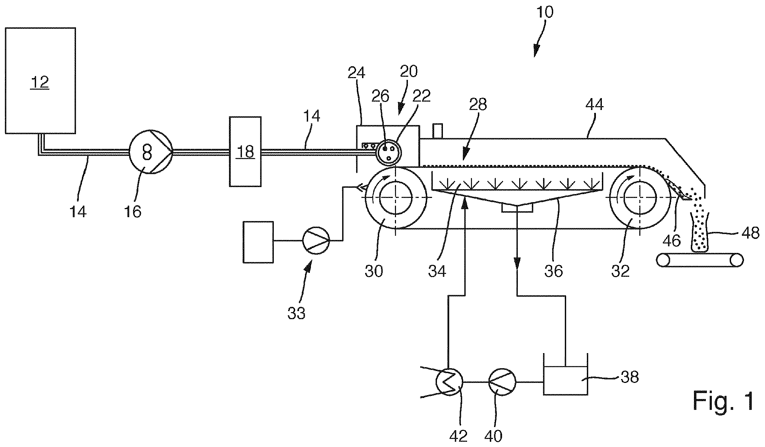

Die schematische Darstellung der Fig. 1 zeigt eine Vorrichtung 10 zum Herstellen von L-Menthol in fester Form. Eine Menthol-Schmelze wird mittels einer lediglich schematisch dargestellten Vorrichtung 12 erzeugt. Über eine Leitung 14 gelangt die Menthol-Schmelze zu einer Pumpe 16 und wird dann über einen Schabekühler 18 geleitet, in dem Impfkristalle in einer Menge von etwa 10 Gew. % der Menthol-Schmelze erzeugt werden. Der Schabekühler 18 weist in bekannter Weise wenigstens eine gekühlte Oberfläche auf, an der die Menthol-Schmelze teilweise kristallisiert. Gemäß der Erfindung wird die Temperatur dieser gekühlten Oberfläche oder Schabefläche in einem kleinen Temperaturbereich zwischen 26° und 32° gehalten. Diese Kristalle werden abgeschabt und somit der übrigen Menthol-Schmelze hinzugefügt. Die Leitung 14, die beheizt bzw. gekühlt sein kann, führt dann weiter zu einem Tropfenformer 20, der eine rotierende Außentrommel 22 aufweist und der unterhalb einer Haube 24 angeordnet ist. Ein Raum unterhalb der Haube 24 kann klimatisiert bzw. auf einer konstanten Temperatur gehalten werden. Dies ist gerade bei der Pastillierung von L-Menthol vorteilhaft, da dadurch auch die Außenflächen der rotierenden Trommel 22 des Tropfenformers auf einer konstanten Temperatur gehalten werden können.

Die rotierende Außentrommel 22 umgibt einen feststehenden Kern des Tropfenformers, in dem eine in Fig. 1 nicht dargestellte Düsenleiste sowie mehrere Kanäle 26 zum Durchleiten von Kühlflüssigkeit vorgesehen sind. Mittels dieser Kanäle bzw. der darin entlanggeführten Kühlflüssigkeit wird der Tropfenformer 20 in einem eng tolerierten Temperaturbereich gehalten. Dieser Temperaturbereich ist so ausgelegt, dass sich innerhalb des Tropfenformers 20 keine oder nur unwesentliche Mengen an Ablagerungen aus kristallisierter L-Menthol-Schmelze bilden und andererseits die L-Menthol-Schmelze innerhalb des Tropfenformers 20 eine so hohe Viskosität aufweist, dass die erzeugten Tropfen beim Auftreffen auf ein unterhalb des Tropfenformers 20 angeordnetes Kühlband nicht sofort auseinanderlaufen, sondern eine kugelähnliche Form beibehalten.

Das Kühlband 28 ist als endloses, umlaufendes Band ausgeführt und ist über zwei Umlenktrommeln 30, 32 geführt. Der Tropfenformer 20 ist oberhalb der in Fig. 1 linken Umlenktrommel 30 angeordnet. Von der rotierenden Außentrommel 22 des Tropfenformers 20 ausgehende Menthol-Schmelze-Tropfen werden somit auf dem Obertrum des Kühlbands 28 abgelegt und dann in Fig. 1 nach rechts abtransportiert. Unterhalb des Obertrums des Kühlbands 28 sind Sprühdüsen 32 angeordnet, die eine Kühlflüssigkeit, insbesondere Wasser, gegen eine Unterseite des Kühlbands 28 sprühen. Das Kühlband 28 besteht vorteilhafterweise aus Stahl, insbesondere Edelstahl, und sorgt dadurch für eine rasche Abkühlung und Verfestigung der vom Tropfenformer 20 auf dem Kühlband 28 abgelegten Tropfen der Menthol-Schmelze. Das von den Sprühdüsen 32 gegen die Unterseite des Kühlbands 28 gesprühte Kühlwasser wird in einer Wanne 36 aufgefangen und einem Sammelbehälter 38 zugeführt. Aus dem Sammelbehälter 38 wird die Kühlflüssigkeit mittels einer Pumpe 40 abgezogen, über einen Wärmetauscher 42 geleitet, der die gewünschte Temperatur der Kühlflüssigkeit einstellt und von dort aus wieder zu den Sprühdüsen 34 geleitet.

Das Obertrum des Kühlbands 28, auf dem die Menthol-Schmelze-Tropfen transportiert werden, ist in seinem Bereich oberhalb der in Fig. 1 linken Umlenktrommel 30 von der Haube 24 umgeben. Stromabwärts der Haube 24 schließt sich eine weitere Haube 44 an, die sich noch über den Bereich der in Fig. 1 rechten Umlenktrommel 32 hinaus erstreckt. Dadurch kann zusätzlich zur Kühlung des Kühlbands 28 auch oberhalb des Kühlbands 28 eine definierte Temperatur eingestellt werden, so dass die von dem Tropfenformer 20 auf dem Kühlband 28 abgelegten Menthol-Schmelze-Tropfen rasch und in der gewünschten Konfiguration erstarren, während sich die Menthol-Schmelze-Tropfen auf dem Kühlband 28 vom Bereich der linken Umlenktrommel 30 zum Bereich der rechten Umlenktrommel 32 bewegen.

An der rechten Umlenktrommel 32 werden die dann verfestigten L-Menthol-Pastillen mittels eines Abnahmemessers 46 abgenommen und beispielsweise in Säcke 48 verpackt.

Eine Vorrichtung 33 dient zum Aufbringen eines Trennmittels stromaufwärts des Aufgabepunkts der Menthol-Schmelze-Tropfen, um ein starkes Anhaften der Tropfen auf dem Kühlband zu vermeiden.

Aufgrund der kugelähnlichen Form der L-Menthol-Pastillen weisen diese eine im Verhältnis zum Volumen geringe Oberfläche auf. Darüber hinaus weisen die erzeugten L-Menthol-Pastillen eine vergleichsweise glatte Oberfläche auf, die insbesondere keinerlei Bruchkanten aufweist. Durch die geringe und dabei glatte Oberfläche werden die Lagereigenschaften der erzeugten L-Menthol-Pastillen äußerst positiv beeinflusst und die Neigung der Pastillen zum Verbacken ist stark reduziert.

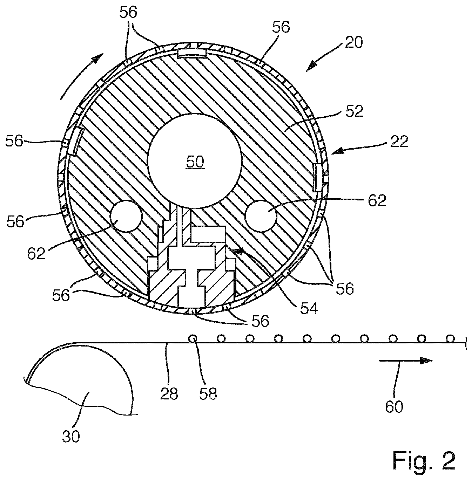

Fig. 2 zeigt eine schematische Schnittansicht des Tropfenformers 20 der Fig. 1 mit dem unterhalb des Tropfenformers 20 angeordneten Kühlband 28, das lediglich abschnittsweise dargestellt ist. Die mit Impfkristallen versehene Menthol-Schmelze wird einem zentralen Zuführkanal 50 im Kern 52 des Tropfenformers 20 zugeführt. Der Kern 52 ist dabei als massiver Kreiszylinder ausgeführt, dessen Außendurchmesser geringfügig kleiner ist als der Innendurchmesser der gelochten Außentrommel 22. Die innerhalb des Zuführkanals 50 zugeführte Menthol-Schmelze wird dann in eine Düsenleiste 54 weiterbefördert, die an der Innenseite der gelochten Außentrommel 22 anliegt. Mittels der Düsenleiste 54 wird die Menthol-Schmelze durch Öffnungen 56 in der gelochten Außentrommel 22 gedrückt. Jedes Mal, wenn eine der Öffnungen 56 unterhalb der Düsenleiste vorbeiläuft, wird somit ein Menthol-Schmelze-Tropfen 58 erzeugt, der dann, siehe Fig. 2, unterhalb der Düsenleiste 54 und der gelochten Außentrommel 22 auf der Oberseite des Kühlbands 28 abgelegt wird. Das Obertrum des Kühlbands 28 bewegt sich, wie in Fig. 2 durch den Pfeil 60 angedeutet ist, von links nach rechts und transportiert somit die Menthol-Schmelze-Tropfen 58 aus dem Bereich unterhalb des Tropfenformers 20 heraus. Dadurch können nacheinander Reihen von Menthol-Schmelze-Tropfen 58 auf dem Kühlband 28 abgelegt werden, wie in Fig. 2 dargestellt ist.

Der Kern 52 des Tropfenformers 20 weist, wie in Fig. 2 dargestellt ist, zwei Kanäle 62 für Kühlflüssigkeit auf. Diese Kanäle sind benachbart zu dem zentralen Zuführkanal 50 und der Düsenleiste 54 angeordnet. Die Kühlflüssigkeit innerhalb dieser Kanäle 62 hält den Kern 52 in einem eng tolerierten Temperaturbereich. Dadurch kann die mit Impfkristallen versehene Menthol-Schmelze innerhalb des Kanals 50 sowie innerhalb der Düsenleiste 54 auf eine Viskosität eingestellt werden, die dafür sorgt, dass die auf dem Kühlband 28 abgelegten Menthol-Schmelze-Tropfen 58 eine kugelähnliche Form aufweisen. Gleichzeitig ist der Temperaturbereich des Kerns 52 so ausgelegt, dass Ablagerungen innerhalb des Kanals 50 und der Düsenleiste 54 weitgehend vermieden werden.

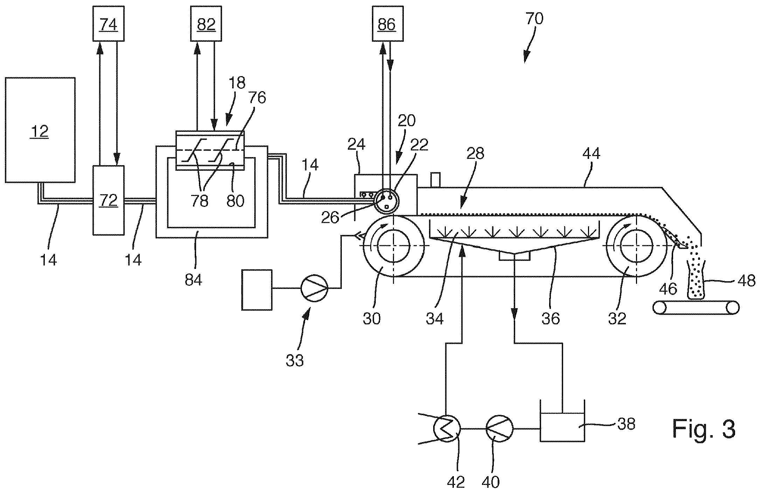

Die schematische Darstellung der Fig. 3 zeigt eine erfindungsgemäße Vorrichtung 70 gemäß einer weiteren Ausführungsform. Bestandteile der Vorrichtung 70, die identisch zu Bestandteilen der Vorrichtung 10 der Fig. 1 sind, werden dabei nicht erneut beschrieben.

Im Unterschied zur Vorrichtung 10 der Fig. 1 führt die Leitung 14 stromabwärts der Vorrichtung 12 zum Erzeugen einer Mentholschmelze zunächst zu einem Vorkühler 72. In diesem Vorkühler 72 wird die Mentholschmelze gekühlt und in einen für den Betrieb des dann stromabwärts folgenden Schabekühlers 18 vorteilhaften Temperaturbereich gebracht. Der Vorkühler 72 ist mit einer Temperiereinheit 74 versehen, über die flüssiges Kühlmittel zu dem Vorkühler 72 geführt und wieder von diesem abgezogen wird. Die Temperatur in diesem Kühlmittelkreislauf zwischen Temperiereinheit 74 und Vorkühler 72 wird dabei in der Temperiereinheit 74 mittels heißer Kühlflüssigkeit, insbesondere Wasser, oder Dampf, insbesondere Wasserdampf, und kalter Kühlflüssigkeit, insbesondere Wasser, eingestellt. Auf diese Weise kann sehr schnell auf veränderliche Kühlflüssigkeitstemperaturen im Rücklauf von dem Vorkühler 72 zur Temperiereinheit 74 reagiert werden und die Temperatur des Vorkühlers 72 kann innerhalb eines eng tolerierten Temperaturbereichs gehalten werden.

Der Schabekühler 18 ist in Fig. 3 schematisch dargestellt und weist eine Welle 76 auf, die mit Schabern 78 versehen sind und die die kreiszylindrische Innenfläche 80 des Schabekühlers 18 abschaben. Diese kreiszylindrische Innenfläche 80 wird auf eine Temperatur zwischen 26° Celsius und 32° Celsius gekühlt, um eine Kristallisation der Mentholschmelze in der gewünschten Kristallkonfiguration zu ermöglichen. Die Temperatur der Innenfläche 80 wird dabei mittels einer weiteren Temperiereinheit 82 eingestellt, von der flüssiges Kühlmittel zum Schabekühler 18 und vom Schabekühler 18 zur Temperiereinheit 82 in einem Kreislauf geführt wird. Die Einstellung der Temperatur des flüssigen Kühlmittels in der Temperiereinheit 82 erfolgt dabei mittels heißer Kühlflüssigkeit, insbesondere Wasser, oder Dampf, insbesondere Wasserdampf, sowie kalter Kühlflüssigkeit, insbesondere Wasser. Dadurch ist es möglich, die Temperatur der Schabefläche 80 im Schabekühler 18 innerhalb des Temperaturbereichs zwischen 26° Celsius und 32° Celsius zu halten.

Die Menge der Kühlflüssigkeit, die dabei zwischen der Temperiereinheit 82 und dem Schabekühler 18 im Kreis gepumpt und temperiert wird, beträgt dabei mindestens das 10-fache der Mentholschmelzenmenge, die durch den Schabekühler 18 gefördert wird. Auf diese Weise kann ein sehr kleiner Temperaturbereich am Schabekühler sichergestellt werden. Auch die Kreisläufe zwischen der Temperiereinheit 74 und dem Vorkühler 72 sowie zwischen der Temperiereinheit 86 und dem Tropfenformer 20 können in entsprechender Weise ausgebildet sein.

Die Mentholschmelze wird stromabwärts des Vorkühlers 72 über die Leitung 14 in eine Kreislaufleitung 84 eingespeist, die die Mentholschmelze zum Schabekühler 18 und vom Schabekühler 18 wieder zur Einströmseite des Schabekühlers 18 führt. Aus dieser Kreislaufleitung 84 zweigt dann auch der weitere Verlauf der Leitung 14 ab, die dann zum Tropfenformer 20 führt. Die Menge der Mentholschmelze, die in der Kreislaufleitung 84 gefördert wird, beträgt dabei mindestens das 10-fache der Menge an Mentholschmelze, die vom Vorkühler 72 über die Leitung 14 zugeführt wird bzw. die über die Leitung 14 in Richtung des Tropfenformers 20 abgeführt wird. Auf diese Weise passiert die Mentholschmelze den Schabekühler 18 mehrfach und es können die gewünschten hohen Verweilzeiten und dadurch die Erzeugung einer großen Menge an Impfkristallen, insbesondere zwischen 30 Gew.-% und 40 Gew.-% der Mentholschmelze, erzeugt werden.

Die Pastillierung der Mentholschmelze im Tropfenformer 20 wurde bereits beschrieben. Um den Tropfenformer 20 auf einer gewünschten Temperatur zu halten, ist dem Tropfenformer 20 eine eigene Temperiereinheit 86 zugeordnet, die in gleicher Weise wie die Temperiereinheiten 74, 82 arbeitet.

## Patentansprüche

1. Verfahren zum Herstellen von L-Menthol in fester Form, **gekennzeichnet durch** folgende Schritte:
- Bereitstellen einer Mentholschmelze,
- Zuführen der Schmelze zu einem Tropfenformer (20) mit einer rotierenden, gelochten Außentrommel (22) und einer an der Innenseite der Außentrommel (22) anliegenden, feststehenden Düsenleiste (54),
- Ablegen von mit dem Tropfenformer (20) erzeugten Schmelzetropfen (58) auf einem umlaufenden Kühlband (28),
- Verfestigen der Schmelzetropfen (58) während des Transports auf dem Kühlband (28) zu L-Menthol-Pastillen und
- Abnehmen der Pastillen im Bereich einer Umlenktrommel (32) für das Kühlband (28).

2. Verfahren nach Anspruch 1, **gekennzeichnet durch** Leiten der Mentholschmelze durch einen Schabekühler (18) vor dem Zuführen der Schmelze zu dem Tropfenformer (20) zum Erzeugen von Impfkristallen.

3. Verfahren nach Anspruch 2, **gekennzeichnet durch** Erzeugen von mehr als 10 Gewichtsprozent, insbesondere 20 Gewichtsprozent, Impfkristallen in der Mentholschmelze im Schabekühler (18).

4. Verfahren nach Anspruch 3, **gekennzeichnet durch** Erzeugen von 30 bis 40 Gewichtsprozent Impfkristallen in der Mentholschmelze im Schabekühler (18).

5. Verfahren nach wenigstens einem der vorstehenden Ansprüche 2 bis 4, **gekennzeichnet durch** Einstellen einer Temperatur an den Schabeflächen des Schabekühlers (18) in einem Bereich zwischen 26°Celsius und 32°Celsius.

6. Verfahren nach wenigstens einem der Ansprüche 2 bis 5, **gekennzeichnet durch** Fördern der Mentholschmelze in einem Kreislauf durch den Schabekühler (18), so dass die Mentholschmelze den Schabekühler (18) mehrfach durchläuft.

7. Verfahren nach Anspruch 6, **dadurch gekennzeichnet, dass** eine Fördermenge im Kreislauf durch den Schabekühler (18) mindestens das Zehnfache, insbesondere das Zwanzigfache, einer Fördermenge vom Schabekühler (18) zum Tropfenformer (20) beträgt.

8. Verfahren nach wenigstens einem der vorstehenden Ansprüche 2 bis 7, **dadurch gekennzeichnet, dass** der Schabekühler (18) mittels flüssigem Kühlmittel, insbesondere Wasser, gekühlt wird, wobei eine Kühlmittelmenge, die durch den Schabekühler gefördert wird, mindestens das Zehnfache der Mentholschmelzenmenge beträgt, die durch den Schabekühler (18) gefördert wird.

9. Verfahren nach Anspruch 8, **gekennzeichnet durch** Einstellen einer Temperatur des flüssigen Kühlmittels in einer Temperiereinheit (82) unter Verwendung von heißer Kühlflüssigkeit oder Dampf und kalter Kühlflüssigkeit.

10. Verfahren nach wenigstens einem der vorstehenden Ansprüche, **gekennzeichnet durch** Leiten der Mentholschmelze durch einen Vorkühler (72) vor dem Zuführen der Schmelze zu dem Tropfenformer (20).

11. Verfahren nach Anspruch 10, **gekennzeichnet durch** Leiten der Mentholschmelze durch einen Schabekühler (18) zum Erzeugen von Impfkristallen nach dem Leiten der Mentholschmelze durch den Vorkühler (72) und vor dem Zuführen der Schmelze zu dem Tropfenformer (20).

12. Verfahren nach wenigstens einem der vorstehenden Ansprüche, **gekennzeichnet durch** Einstellen einer Temperatur im Tropfenformer (20) oberhalb des Schmelzpunkts der Mentholschmelze, so dass eine Kristallisation der Mentholschmelze im Tropfenformer (20) nur in sehr geringem Ausmaß erfolgt und Ablagerungen vermieden werden.

13. Vorrichtung zum Herstellen von L-Menthol in fester Form, **gekennzeichnet durch** Mittel (12) zum Bereitstellen einer Mentholschmelze, einem Tropfenformer (20) mit einer rotierenden, gelochten Außentrommel (22) und einer an der Innenseite der Außentrommel (22) anliegenden, feststehenden Düsenleiste (54), sowie einem unterhalb der Außentrommel (22) angeordneten umlaufenden Kühlband (28) und durch einen Schabekühler (18) zum Erzeugen von Impfkristallen in der Mentholschmelze, wobei der Schabekühler (18) stromaufwärts des Tropfenformers (20) angeordnet ist.

14. Vorrichtung nach Anspruch 13, **dadurch gekennzeichnet, dass** der Schabekühler (18) eine kreiszylindrische, gekühlte Innenfläche (80) aufweist, die von auf einer rotierenden Welle (76) angeordneten Schabern (78) überstrichen wird.

15. Vorrichtung nach einem der Ansprüche 13 bis 14, **dadurch gekennzeichnet, dass** stromaufwärts des Schabekühlers (18) ein Vorkühler (72) für die Mentholschmelze angeordnet ist.

16. Vorrichtung nach Anspruch 15, **dadurch gekennzeichnet, dass** der Vorkühler (72), der Schabekühler (18) und/oder der Tropfenformer (20) mit separaten Kühlflüssigkeitskreisläufen versehen sind.

17. Vorrichtung nach Anspruch 16, **dadurch gekennzeichnet, dass** den Kühlkreisläufen jeweils separate Temperiereinheiten (74, 82, 86) zugeordnet sind, wobei jede Temperiereinheit (74, 82, 86) eine Heizvorrichtung zum Bereitstellen von heißer Kühlflüssigkeit oder Dampf und eine Kühvorrichtung zum Bereitstellen von kalter Kühlflüssigkeit aufweist.

## Claims

1. Method for producing L-menthol in solid form, **characterized by** the following steps:
- providing a menthol melt,
- feeding the melt to a drop former (20) having a rotating, perforated outer drum (22) and a fixed nozzle strip (54) adjacent to the inside of the outer drum (22),
- depositing melt drops (58) produced by the drop former (20) onto a continuous cooling belt (28),
- solidifying the melt drops (58) during transport on the cooling belt (28) to form L-menthol pellets, and
- taking the pellets off in the region of a deflection drum (32) for the cooling belt (28).

2. Method according to claim 1, **characterized by** conveying the menthol melt through a scraping cooler (18) before feeding the melt to the drop former (20) in order to produce seed crystals.

3. Method according to claim 2, **characterized by** producing more than 10% by weight, in particular 20% by weight, of seed crystals in the menthol melt in the scraping cooler (18).

4. Method according to claim 3, **characterized by** producing from 30 to 40% by weight of seed crystals in the menthol melt in the scraping cooler (18).

5. Method according to at least one of the preceding claims 2 to 4, **characterized by** setting a temperature at the scraping surfaces of the scraping cooler (18) in a range from 26° Celsius to 32° Celsius.

6. Method according to at least one of the claims 2 to 5, **characterized by** conveying the menthol melt in a circuit through the scraping cooler (18) so that the menthol melt passes through the scraping cooler (18) a plurality of times.

7. Method according to claim 6, **characterized in that** an amount conveyed in the circuit through the scraping cooler (18) is at least ten times, in particular twenty times, an amount conveyed from the scraping cooler (18) to the drop former (20).

8. Method according to at least one of the preceding claims 2 to 7, **characterized in that** the scraping cooler (18) is cooled by means of a liquid coolant, in particular water, with an amount of coolant conveyed through the scraping cooler being at least ten times the amount of menthol melt conveyed through the scraping cooler (18).

9. Method according to claim 8, **characterized by** setting a temperature of the liquid coolant in a temperature control unit (82) using hot cooling liquid or steam and cold cooling liquid.

10. Method according to at least one of the preceding claims, **characterized by** conveying the menthol melt through a precooler (72) before feeding the melt to the drop former (20).

11. Method according to claim 10, **characterized by** conveying the menthol melt through a scraping cooler (18) in order to produce seed crystals after conveying the menthol melt through the precooler (72) and before feeding the melt to the drop former (20).

12. Method according to at least one of the preceding claims, **characterized by** setting a temperature in the drop former (20) above the melting point of the menthol melt, so that crystallization of the menthol melt in the drop former (20) occurs to only a very small extent and deposits are avoided.

13. Device for producing L-menthol in solid form, **characterized by** means (12) for providing a menthol melt, a drop former (20) having a rotating, perforated outer drum (22) and a fixed nozzle strip (54) adjacent to the inside of the outer drum (22) and also a continuous cooling belt (28) arranged underneath the outer drum (22), and by a scraping cooler (18) for producing seed crystals in the menthol melt, with the scraping cooler (18) being arranged upstream of the drop former (20).

14. Device according to claim 13, **characterized in that** the scraping cooler (18) has a circular cylindrical, cooled interior surface (80) which is scraped by scrapers (78) arranged on a rotating shaft (76).

15. Device according to any of claims 13 to 14, **characterized in that** a precooler (72) for the menthol melt is arranged upstream of the scraping cooler (18).

16. Device according to claim 15, **characterized in that** the precooler (72), the scraping cooler (18) and/or the drop former (20) are provided with separate cooling liquid circuits.

17. Device according to claim 16, **characterized in that** separate temperature control units (74, 82, 86) are assigned to each of the cooling circuits, wherein each temperature control unit (74, 82, 86) has a heating device for providing hot cooling liquid or steam and a cooling device for providing cold cooling liquid.

## Revendications

1. Procédé de fabrication de L-menthol sous forme solide, **caractérisé par** les étapes suivantes :
- la préparation d'une masse fondue de menthol,
- l'introduction de la masse fondue dans un dispositif de formation de gouttes (20) comprenant un tambour extérieur perforé rotatif (22) et une barre de buses fixe (54) disposée sur le côté intérieur du tambour extérieur (22),
- le dépôt des gouttes de masse fondue (58) formées avec le dispositif de formation de gouttes (20) sur une bande de refroidissement en circulation (28),
- la solidification des gouttes de masse fondue (58) pendant le transport sur la bande de refroidissement (28) en pastilles de L-menthol, et
- le retrait des pastilles dans la zone d'un tambour de retour (32) pour la bande de refroidissement (28).

2. Procédé selon la revendication 1, **caractérisé par** l'acheminement de la masse fondue de menthol au travers d'un refroidisseur à raclage (18) avant l'introduction de la masse fondue dans le dispositif de formation de gouttes (20) pour la formation de germes cristallins.

3. Procédé selon la revendication 2, **caractérisé par** la formation de plus de 10 pour cent en poids, notamment de 20 pour cent en poids, de germes cristallins dans la masse fondue de menthol dans le refroidisseur à raclage (18).

4. Procédé selon la revendication 3, **caractérisé par** la formation de 30 à 40 pour cent en poids de germes cristallins dans la masse fondue de menthol dans le refroidisseur à raclage (18).

5. Procédé selon au moins l'une quelconque des revendications 2 à 4 précédentes, **caractérisé par** l'ajustement d'une température sur les surfaces de raclage du refroidisseur à raclage (18) dans une plage comprise entre 26 °C et 32 °C.

6. Procédé selon au moins l'une quelconque des revendications 2 à 5, **caractérisé par** le transport de la masse fondue de menthol dans un circuit au travers du refroidisseur à raclage (18), de telle sorte que la masse fondue de menthol traverse à plusieurs reprises le refroidisseur à raclage (18).

7. Procédé selon la revendication 6, **caractérisé en ce qu'**une quantité transportée dans le circuit au travers du refroidisseur à raclage (18) est d'au moins dix fois, notamment de vingt fois, une quantité transportée depuis le refroidisseur à raclage (18) vers le dispositif de formation de gouttes (20).

8. Procédé selon au moins l'une quelconque des revendications 2 à 7 précédentes, **caractérisé en ce que** le refroidisseur à raclage (18) est refroidi au moyen d'un réfrigérant liquide, notamment d'eau, une quantité de réfrigérant qui est transportée au travers du refroidisseur à raclage étant d'au moins dix fois la quantité de masse fondue de menthol qui est transportée au travers du refroidisseur à raclage (18).

9. Procédé selon la revendication 8, **caractérisé par** l'ajustement d'une température du réfrigérant liquide dans une unité de conditionnement en température (82) en utilisant un liquide réfrigérant chaud ou de la vapeur d'eau et un liquide réfrigérant froid.

10. Procédé selon au moins l'une quelconque des revendications précédentes, **caractérisé par** l'acheminement de la masse fondue de menthol au travers d'un pré-refroidisseur (72) avant l'introduction de la masse fondue dans le dispositif de formation de gouttes (20).

11. Procédé selon la revendication 10, **caractérisé par** l'acheminement de la masse fondue de menthol au travers d'un refroidisseur à raclage (18) pour la formation de germes cristallins après l'acheminement de la masse fondue de menthol au travers du pré-refroidisseur (72) et avant l'introduction de la masse fondue dans le dispositif de formation de gouttes (20).

12. Procédé selon au moins l'une quelconque des revendications précédentes, **caractérisé par** l'ajustement d'une température dans le dispositif de formation de gouttes (20) supérieure au point de fusion de la masse fondue de menthol, de telle sorte qu'une cristallisation de la masse fondue de menthol dans le dispositif de formation de gouttes (20) n'ait lieu qu'en une mesure très faible et que des dépôts soient évités.

13. Dispositif pour la fabrication de L-menthol sous forme solide, **caractérisé par** des moyens (12) pour la préparation d'une masse fondue de menthol, un dispositif de formation de gouttes (20) comprenant un tambour extérieur perforé rotatif (22) et une barre de buses fixe (54) disposée sur le côté intérieur du tambour extérieur (22), ainsi qu'une bande de refroidissement (28) en circulation agencée en dessous du tambour extérieur (22), et par un refroidisseur à raclage (18) pour la formation de germes cristallins dans la masse fondue de menthol, le refroidisseur à raclage (18) étant agencé en amont du dispositif de formation de gouttes (20).

14. Dispositif selon la revendication 13, **caractérisé en ce que** le refroidisseur à raclage (18) comprend une surface intérieure cylindrique circulaire refroidie (80), qui est balayée par des racleurs (78) agencés sur un arbre rotatif (76).

15. Dispositif selon l'une quelconque des revendications 13 à 14, **caractérisé en ce qu'**un pré-refroidisseur (72) pour la masse fondue de menthol est agencé en amont du refroidisseur à raclage (18).

16. Dispositif selon la revendication 15, **caractérisé en ce que** le pré-refroidisseur (72), le refroidisseur à raclage (18) et/ou le dispositif de formation de gouttes (20) sont munis de circuits de liquide réfrigérant séparés.

17. Dispositif selon la revendication 16, **caractérisé en ce que** des unités de conditionnement en température séparées (74, 82, 86) sont attribuées à chacun des circuits réfrigérants, chaque unité de conditionnement en température (74, 82, 86) comprenant un dispositif de chauffage pour la préparation d'un liquide réfrigérant chaud ou de vapeur d'eau et un dispositif de refroidissement pour la préparation d'un liquide réfrigérant froid.
